(19) Europäisches Patentamt
European Patent Office
Office européen des brevets

(11) **EP 4 291 200 B1**

(12) **EUROPEAN PATENT SPECIFICATION**

(45) Date of publication and mention
of the grant of the patent:
**07.08.2024 Bulletin 2024/32**

(51) International Patent Classification (IPC):
*A61K 31/7036* (2006.01)    *A61K 31/496* (2006.01)
*A61K 38/14* (2006.01)    *A61L 27/12* (2006.01)
*A61P 31/04* (2006.01)    *A61K 45/06* (2006.01)
*A61L 27/02* (2006.01)    *A61L 27/50* (2006.01)
*A61L 27/54* (2006.01)    *A61L 27/58* (2006.01)

(21) Application number: **23707772.2**

(52) Cooperative Patent Classification (CPC):
(C-Sets available)
**A61P 31/04; A61K 31/496; A61K 31/7036;
A61K 38/14; A61K 45/06; A61L 27/025;
A61L 27/12; A61L 27/50; A61L 27/54; A61L 27/58;**
A61L 2300/406; A61L 2300/45; A61L 2400/06;
A61L 2430/02                    (Cont.)

(22) Date of filing: **03.03.2023**

(86) International application number:
**PCT/EP2023/055402**

(87) International publication number:
**WO 2023/166161 (07.09.2023 Gazette 2023/36)**

(54) **NOVEL COMBINATION TREATMENT REGIMEN OF BACTERIAL INFECTIONS**

NEUARTIGES KOMBINATIONSBEHANDLUNGSSCHEMA FÜR BAKTERIELLE INFEKTIONEN

NOUVEAU RÉGIME DE TRAITEMENT COMBINÉ D'INFECTIONS BACTÉRIENNES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.03.2022 EP 22160214**

(43) Date of publication of application:
**20.12.2023 Bulletin 2023/51**

(73) Proprietor: **BONE SUPPORT AB
223 70 Lund (SE)**

(72) Inventor: **LIDGREN, Lars
298 31 Tollarp (SE)**

(74) Representative: **Guardian
IP Consulting I/S
Diplomvej, Building 381
2800 Kgs. Lyngby (DK)**

(56) References cited:
WO-A1-2019/206677    CN-B- 106 943 629
KR-A- 20160 122 656    US-A1- 2014 234 435

- **ROSSELLA DORATI ET AL: "Biodegradable
Scaffolds for Bone Regeneration Combined with
Drug-Delivery Systems in Osteomyelitis
Therapy", PHARMACEUTICALS, vol. 10, no. 4, 12
December 2017 (2017-12-12), pages 96,
XP055702958, DOI: 10.3390/ph10040096**

**(Cont. next page)**

(52) Cooperative Patent Classification (CPC): (Cont.)

C-Sets
**A61K 31/496, A61K 2300/00;**
**A61K 31/7036, A61K 2300/00;**
**A61K 38/14, A61K 2300/00**

**Description**

**Field of the invention**

Present invention relates to the use of a biphasic material, i.e. a ceramic material in combination with administration of one or more pharmaceutical compounds, such as e.g. two or more pharmaceutical compounds in the treatment of bacterial infections enabling prevention of development of bacterial resistance towards administered pharmaceutical compounds in the context of bacterial infections in bone, such as e.g. deep bone infections.

**Background of the invention**

Deep bone infections (DBI's) are one of the most serious complications in orthopedic surgery. In 2018, at International consensus meeting on musculoskeletal infections, the incidences of infection covering all orthopedic subspecialties reported to range from 0.1% to 30%, at a cost of $17 000-$150000 per patient. Due to an epidemic of road traffic accidents, and the high demand for joint replacement, the incidence of DBI's with high patient morbidity and societal burden will obviously increase.

In the poorly vascularized infected bone tissue, achieving an effective and adequate sustained local concentration of a systemically given antibiotics is a challenge. The current treatment methods like long-term systemic antibiotics following local debridement may also lead to serious toxicities and the selection of antibiotic-resistant bacteria. In addition, bacteria are being sheltered residing within biofilms which further limit treatment efficacy.

In order to achieve high local antibiotics levels at minimum inhibitory concentration (MIC) or minimum biofilm eradication concentration (MBEC), surgeons in the last few decades have used polymethylmethacrylate (PMMA) containing antibiotics for local delivery. However, PMMA as a carrier does not give sustained release and lacks bone regeneration properties. There are recently approved biphasic ceramic carriers achieving high local release of gentamicin or vancomycin reporting good outcome in successful debrided bone infections. No osteoconductive biomaterial however currently contains antibiotics like tetracyclines, and rifampicin considered as cornerstone second defence antibiotics in the treatment of severe longstanding DBI's. Moreover, due to incomplete setting and mechanical properties, PMMA is not suitable as carrier for local delivery of RIF. Commercially available, bi-phasic, bioresorbable Calcium sulphate/hydroxyapatite (CaS/HA) bone void fillers, composed of 60 wt% $\alpha$-calcium sulfate hemihydrate (CaS) and 40 wt% hydroxyapatite (HA) has ability to deliver biologically active molecules, hormones and anti-cancer drugs. Also, various clinical studies have reported their potential as a carrier for antibiotics such as gentamicin (GEN) and vancomycin (VAN) for the treatment of bone infections, with less side effects as compared to the commonly used systemic treatment mode. However, RIF monotherapy is not recommended due to rapid development of bacterial resistance.

With respect to related prior art, the following may be mentioned;

WO 2019/206677 relates to a method of administering one or more pharmaceutical compounds to a subject in need thereof by the aid of a mono or biphasic material, such as e.g. a ceramic material in combination with administration of one or more pharmaceutical compounds.

US 2014/234435 relates to hardenable ceramic bone substitute compositions having improved setting, powders for such compositions and methods for their manufacture and use in medical treatment. More specifically the docment relates to hardenable bone substitute powder and hardenable bone substitute paste with improved setting properties, comprising calcium sulfate and heat-treated hydroxyapatite (passivated HA), which bone substitute is suitable for treatment of disorders of supportive tissue such as bone loss, bone fracture, bone trauma and osteomyelitis.

CN 106943629 relates to a tienam-loaded hydroxyapatite-zymolytic collagen protein nano artificial bone, which is mainly formed by loading tienam to a hydroxyapatite-zymolytic collagen protein nano artificial bone, wherein the hydroxyapatite-zymolytic collagen protein nano artificial bone is obtained in a mode that hydroxyapatite crystals grow in a mineralizing mode along collagen fibers and by biomineralization, a bionic bone is finally formed. The collagen fibers have a characteristic alternately-dark-and-bright periodic strip structure, i.e. a D-Band structure.

KR 2016 0122656 relates to a sustained release drug delivery and a production method thereof. More specifically the document relates to a hydroxyapatite-calcium microsphere as a sustained release drug delivery. To this end, the sustained release drug delivery includes: a microsphere matrix in which a drug and hydroxyapatite having a first degradation speed are dispersed without phase separation; and calcium sulfate particles inserted in the microsphere matrix and having a second decomposition speed which is faster than the first decomposition speed.

Dorati, R., et al., "Biodegradable Scaffolds for Bone Regeneration Combined with Drug-Delivery Systems in Osteomyelitis Therapy", Pharmaceuticals, 2017, 10(4), p. 96 relates to the combination of the local delivery of antibiotic agents with bone regenerative therapy for the treatment of a severe bone infection such as osteomyelitis. The review article relates to a brief explanation of scaffolds for bone regeneration including scaffolds characteristics and types, a focus on severe bone infections (especially osteomyelitis and its treatment), and a literature review of local antibiotic delivery by the combination of scaffolds and drug-delivery systems. Some examples related to published studies on gentamicin sulfate-loaded drug-delivery systems combined with scaffolds are discussed, and future perspectives are highlighted.

## Summary of the invention

The composition for use according to the invention enables an improved therapy overcoming the above mentioned problems and draw-backs.

Specifically, present invention provides for one or more of;

- A reduced or altogether eliminated bacterial resistance or preventing gradual build-up of bacterial resistance against the compounds employed in treating a bacterial infection.
- A reduced or altogether eliminated development of planktonic bacteria and/or bacterial biofilm formation on a surface or a void, particularly the surface of bone and/or a device implanted into bone tissue.
- A local extended release of high concentration of pharmaceutical compounds thereby obtaining a highly effective treatment.
- An improved treatment of bone infections and in particular deep bone infections (DBI).

Thus, present invention provides for an extended release composition for use in treatment of bacterial infections. Specifically, present invention relates to a local extended release composition for use in treatment of bacterial infections or conditions related to bacterial infections. This may allow for a locally high concentration of the employed pharmaceutical compounds which may not be possible by e.g. systemic administration of the same pharmaceutical compounds. This is particularly advantageous when treating e.g. bacterial infections which require a certain MIC (minimum inhibitory concentration) in order to result in a successful treatment.

The materials used according to the invention is hydroxyapatite (HA). This material is used in combination with calcium sulphate (either as a hemi-hydrate and/or di-hydrate). The important feature with the material is that it has to be finely divided into micro or nanoparticles so as to provide a very large effective surface. This may be suitable achieved by any suitable technique known in the art in order to create small particles such as e.g. grinding etc. As mentioned herein, such finely divided particles may be used without addition of further materials, or may be dispersed in a third material. The particles dispersed or mixed with a second or more material, wherein the second or more materials are partly or totally bioabsorbable. Consequently, the material according to the invention comprises at least one first component that is not bioresorbable, or very slowly bioresorbable. This material may optionally comprise at least one second material that is bioresorbable at a higher rate than the first material.

The invention also relates to the use of one or more pharmaceutically active compounds which are antibiotics used in any bone related disease or condition.

Consequently, present invention relates to a composition for use in the treatment or prevention of a bacterial infection in a subject, the composition comprising;

  i) a first material being hydroxyapatite,
  ii) a second material being calcium sulphate,
  iii) two or more pharmaceutical compounds, wherein the at least two pharmaceutical compounds are selected from a combination of vancomycin and rifampicin, or a combination of gentamicin and rifampicin,

to thereby prevent or reduce or eliminate the occurrence of bacterial resistance against said the pharmaceutical compounds.

The bacterial infection may be a bacterial infection in bone tissue. In a particular aspect, present invention relates to treatment of bacterial infection in bone tissue, such as e.g. deep bone infection (DBI).

Moreover, present invention also relates to a method of preparing a composition for use according to the invention, the method comprising the steps of;

  a) providing a particulate first material being hydroxyapatite and a second material being calcium sulphate,
  b) contacting/incubating/soaking two or more pharmaceutical compounds with the material in a) wherein the two or more pharmaceutical compounds are;

    I) vancomycin and rifampicin in combination, or
    II) gentamicin and rifampicin,

  c) separating non-bound pharmaceutical compounds from the material obtained in step b).

**Figures**

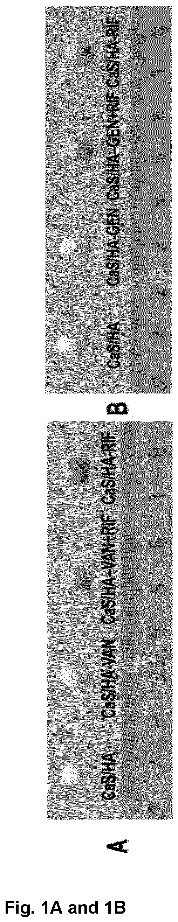

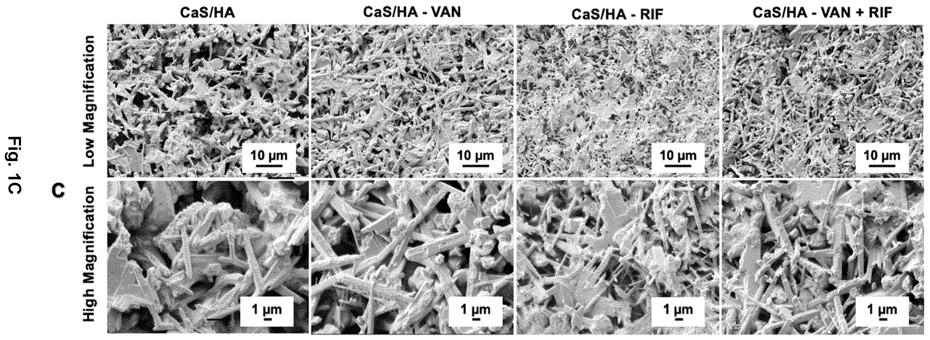

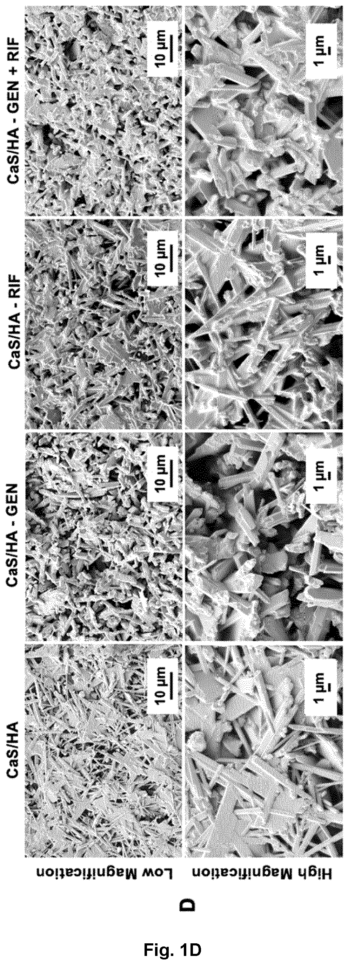

Fig. 1 illustrates the Morphological and Physio-chemical Characterization of CaS/HA-VAN/GEN with or without RIF. (A & B) Hemispherical pellets casted using an elastic mould of 4.8 mm diameter. (C & D) SEM images showing surface characteristics and pore distribution of CaS/-VAN/GEN pellets with or without RIF.

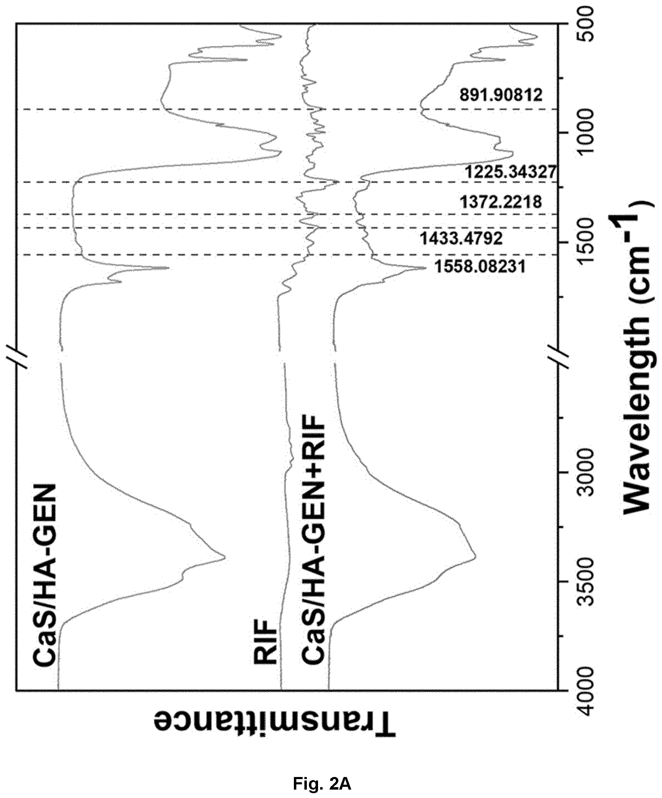

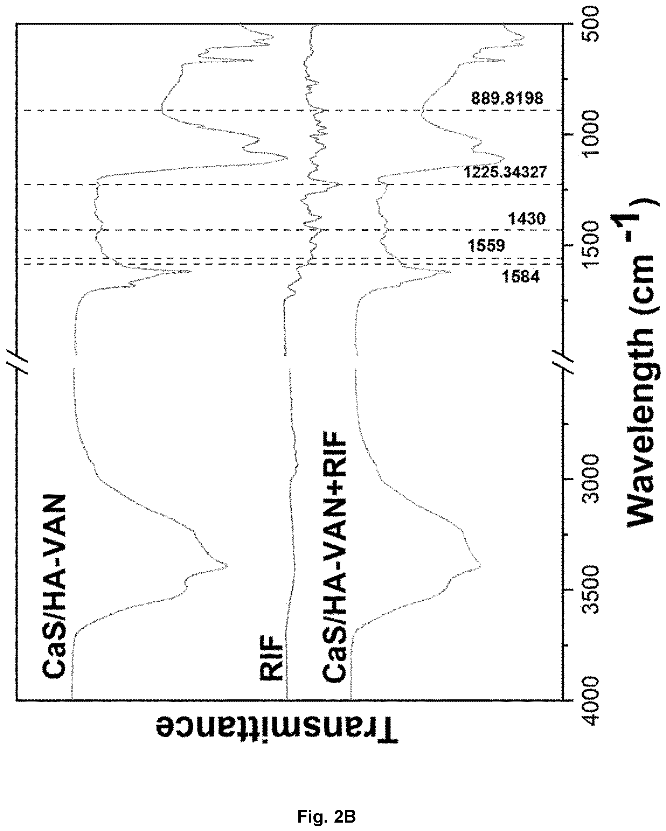

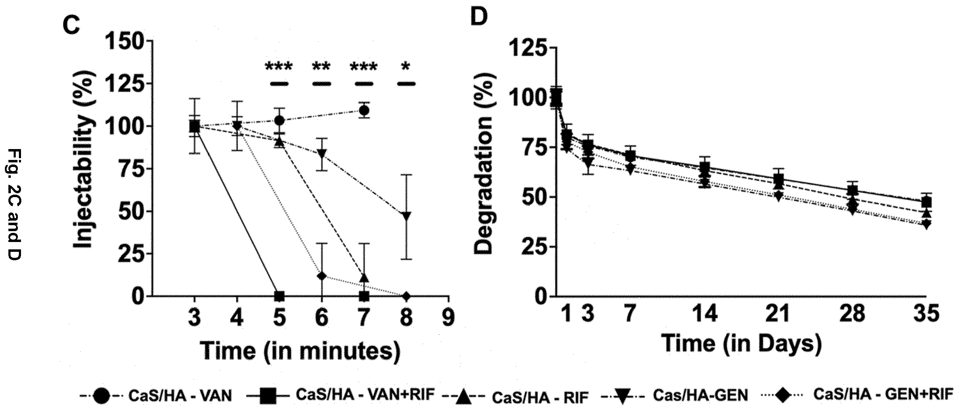

Fig. 2 (A, B) FTIR spectra from CaS/HA-VAN, pure RIF or CaS/HA-VAN+RIF (A), and CaS/HA-GEN, pure RIF or CaS/HA-GEN+RIF (B). (C) Injectability of tested CaS/HA composites. Injectability of CaS/H-VAN/+RIF, CaS/HA-RIF composites were tested at 3, 5 and 7 min, and CaS/HA-GEN/+RIF composites tested at 4, 6 and 8 min. (D) In-vitro material degradation profile of tested CaS/HA-antibiotic composites in PBS at Days 1, 3, 7, 14, 21, 28 and 35. A student t-test was used to compare the injectability and in vitro material degradation profile of CaS/HA having VAN/GEN alone or in combination with RIF. *indicates p<0.05, ** indicates p<0.01, and *** indicates p<0.001.

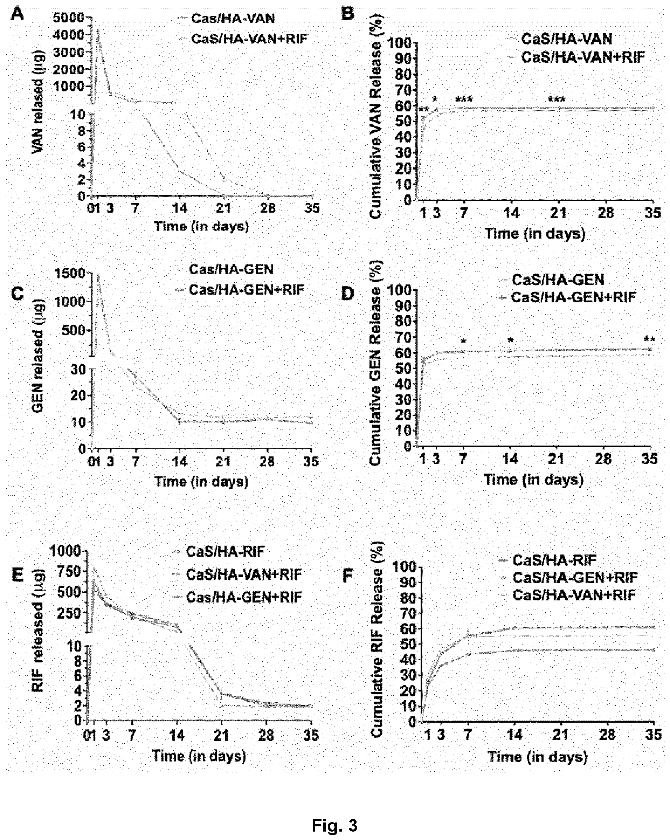

Fig. 3 illustrates the antibiotic release profile. (A-F) Release per day and cumulative release profile of VAN, GEN and RIF from CaS/HA-VAN/GEN with or without RIF at Days 1, 3, 7, 14, 21, 28, and 35. A student t-test was used to compare the cumulative release profile CaS/HA-VAN/GEN alone or in combination with RIF. For comparison of cumulative release profile of RIF, one-way analysis of variance (ANOVA) with Dunn's multiple comparison was used. *indicates p<0.05, ** indicates p<0.01, *** and indicates p<0.001.

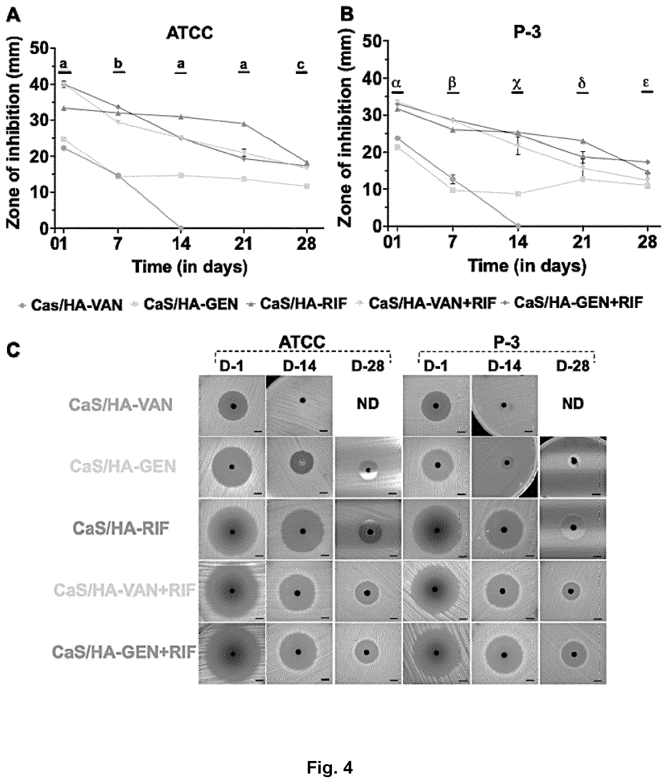

Fig. 4 illustrates the testing of antibacterial efficacy of CaS/HA-antibiotics pellets on planktonic bacteria by continuous Kirby-Bauer disk diffusion assay. A graph data showing the zone of inhibition (ZOI) (in mm) of CaS/HA-antibiotics pellets against (A) *Staphylococcus aureus* ATCC 25923, and (B) *Staphylococcus aureus* clinical strain P3. (C) Muller Hinton agar plates inoculated with *Staphylococcus aureus* ATCC 25923 and clinical strain P3 showing ZOI obtained on D-1, 14, and 28, scale bar = 1.6 cm. For comparison of difference in ZOI between different groups, one-way analysis of variance (ANOVA) with Tukey's multiple comparison was used. "a" in A indicates the statistical significance (p<0.0001) between CaS/HA-VAN/GEN/RIF alone in comparison to the CaS/HA-Van+RIF / CaS/HA-GEN+RIF group. "b" in A indicates the statistical significance of (p<0.0001) between CaS/HA-VAN/GEN alone in comparison to the CaS/HA-VAN/GEN+RIF group, (p<0.001) between CaS/HA-RIF in comparison to the CaS/HA-VAN+RIF group, and (p<0.05) between CaS/HA-RIF in comparison to the CaS/HA-GEN+RIF group. "c" in A indicates the statistical significance of (p<0.0001) between CaS/HA-VAN/GEN alone in comparison to the CaS/HA-VAN/GEN+RIF group, and (p<0.05) between CaS/HA-RIF in comparison to the CaS/HA-VAN+RIF group. "α" in B indicates the statistical significance of (p<0.0001) between CaS/HA-VAN/GEN alone in comparison to the CaS/HA-VAN/GEN+RIF group, and (p<0.01) between CaS/HA-RIF in comparison to the CaS/HA-VAN+RIF group. "β" in B indicates the statistical significance of (p<0.0001) between CaS/HA-VAN/GEN alone in comparison to the CaS/HA-VAN/GEN+RIF group, (p<0.05) between CaS/HA-RIF in comparison to the CaS/HA-VAN+RIF group, and (p<0.01) between CaS/HA-RIF in comparison to the CaS/HA-GEN+RIF group. "χ" in B indicates the statistical significance of (p<0.0001) between CaS/HA-VAN/GEN alone in comparison to the CaS/HA-VAN/GEN+RIF group, and (p<0.05) between CaS/HA-RIF in comparison to the CaS/HA-VAN+RIF group. "δ" in B indicates the statistical significance of (p<0.0001) between CaS/HA-VAN alone in comparison to the CaS/HA-VAN+RIF group, (p<0.001) between CaS/HA-RIF in comparison to the CaS/HA-VAN+RIF group, (p<0.01) between CaS/HA-GEN alone in comparison to the CaS/HA-GEN+RIF group, and (p<0.05) between CaS/HA-RIF in comparison to the CaS/HA-GEN+RIF group. "ε" in B indicates the statistical significance of (p<0.0001) between CaS/HA-VAN alone in comparison to the CaS/HA-VAN+RIF group, (p<0.001) between CaS/HA-GEN in comparison to the CaS/HA-GEN+RIF group. ND-Not done.

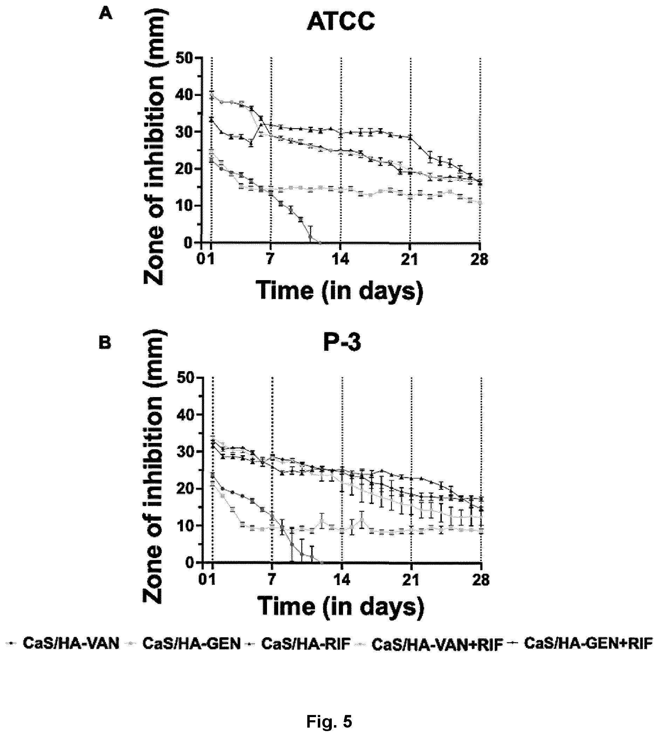

Fig. 5 illustrates the testing of antibacterial efficacy of CaS/HA-antibiotics pellets on planktonic bacteria by continuous Kirby-Bauer disk diffusion assay for 28 days. A graph data showing the zone of inhibition (ZOI) (in mm) of CaS/HA-antibiotics pellets against (A) *Staphylococcus aureus* ATCC 25923, and (B) *Staphylococcus aureus* clinical strain P-3.

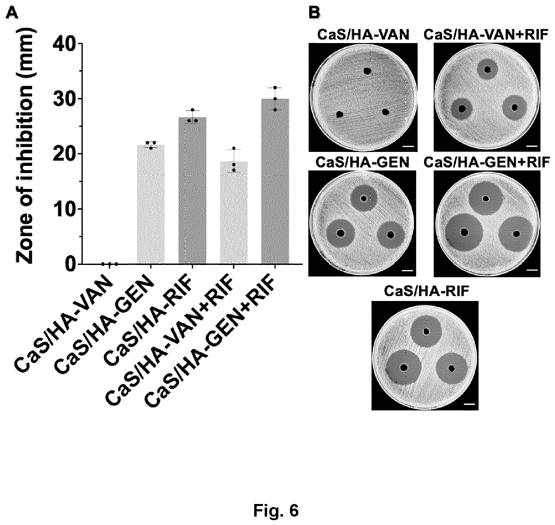

Fig. 6 illustrates the antibacterial effect of pellets after day-35. (A) A graph data showing the antibacterial effect of the pellets placed in PBS for 35 days. (B) Muller Hinton agar plates inoculated with *Staphylococcus aureus* ATCC 25923 showing ZOI, scale bar = 2.6 cm.

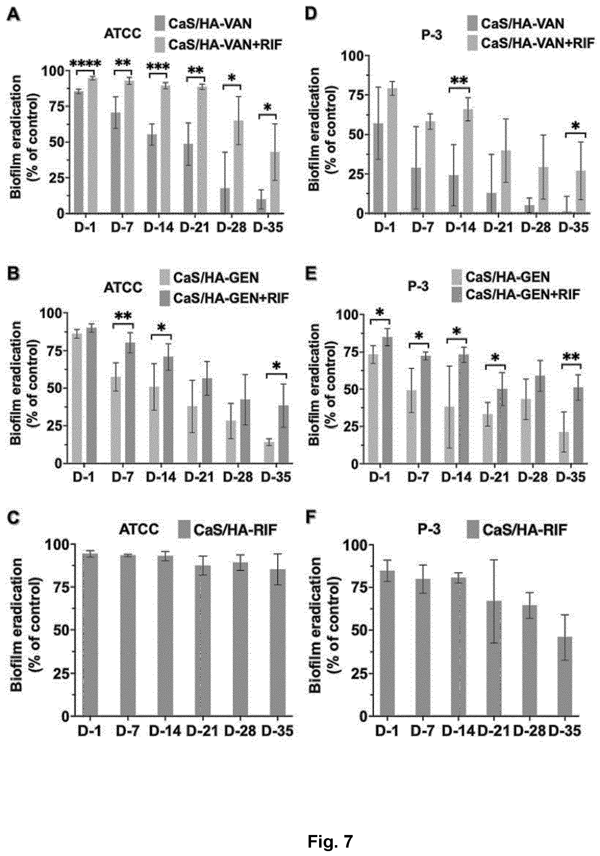

Fig. 7 illustrates the efficacy of antibiotic fractions from D-1, 7, 14, 21, 28 & 35 on preformed biofilms. (A-F) Graphical representation of the *Staphylococcus aureus* ATCC 25923 and clinical strain P3 biofilm destruction obtained by crystal violet staining. A student t-test was used to compare the differences in biofilm destruction between CaS/HA-VAN/GEN with or without RIF. * indicates p<0.05, ** indicates p<0.01, *** indicates p<0.001, and **** indicates p<0.0001.

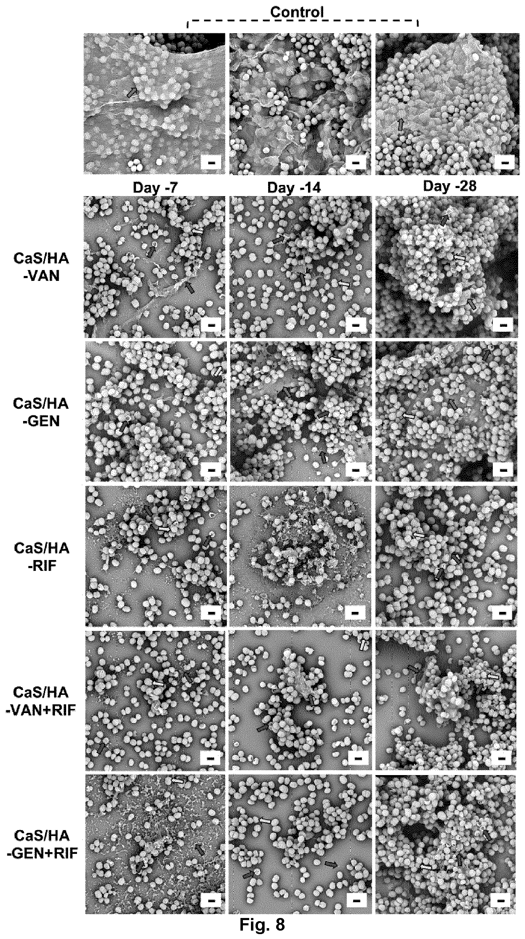

Fig. 8 illustrates the SEM images of *Staphylococcus aureus* ATCC 25923 biofilm destruction. For all tested CaS/HA-antibiotic groups, biofilm destruction and antibacterial effects were evident on day-7 and 14. At day-28, number of planktonic bacteria were increased and biofilm were visible. Green arrow indicates biofilm while violet arrow indicates destructed biofilm. Red arrow indicates dead bacteria. White arrow indicates membrane disruption and extravasation of cytoplasmic components of bacteria. Scale bar = 1 μm.

**Detailed description of the invention**

Present invention relates to the use of one or more materials. The material may is a bi-phasic. In the context of the invention the material has to have at least one component that is not bioresorbable or very slowly bioresorbable and in the context of the invention such material is hydroxyapatite.

By the term "bioresordable" or bioresorbable" is meant a material that can be broken down and absorbed by the body, and thus does not need to be removed manually. Such material may e.g. be integrated by the body, such as e.g. be integrated into bone tissue, or otherwise metabolized by the organism.

According to the invention, the bioresorbable material is apatite or hydroxyapatite (HA) in any configuration or crystal form. Such material is slowly bioresorbable and may eventually be integrated into the bone tissue of the subject. The material may have a wholly or partially crystalline microstructure. Another feature is that the material according to the invention is finely divided into small particles such as e.g. micro or even nanoparticles. As a consequence thereof, the effective surface of a certain amount of such material is very large. It is to be noted that according to the invention the above described material may be denoted as a first material. Merely for sake of completion, hydroxyapatite which is also referred to as hydroxylapatite, has the formula $Ca_5(PO_4)_3(OH)$, but may also be written as $Ca_{10}(PO_4)_6(OH)_2$ to denote that the crystal unit cell comprises two entities. This entity may be denoted or abbreviated as "HA" in present text.

In a particular embodiment, the first material is hydroxyapatite (HA) in any configuration or particle size.

As mentioned above the first material is absorbed very slowly by the organism. Typically, the period of absorption may be in the range of weeks, months and even years, such as e.g. about 2 weeks, such as e.g. about 3 weeks, such as e.g. about 4 weeks, such as e.g. about 5 weeks, such as e.g. about 6 weeks, such as e.g. about 7 weeks, such as e.g. about 2 months, such as e.g. about 3 months, such as e.g. about 4 months, such as e.g. about 6 months, such as e.g. about 8 months, such as e.g. about 10 months, such as e.g. about 12 months, such as e.g. about 18 months, such as e.g. about 2 years, or such as e.g. several years etc. after administration to the subject. In one aspect, the first material may not be absorbed at all by the organism.

In one aspect, the first material is absorbed very slowly by the organism, such as e.g. about 6 months, such as e.g. about 8 months, such as e.g. about 10 months, such as e.g. about 12 months, such as e.g. about 18 months, such as e.g. about 2 years, or between about 6 months to about 2 years after administration to the subject. In a further aspect the first material is absorbed very slowly by the organism, such as e.g. about 6 months to about 2 years.

According to the invention, the first material is mixed with a second material. According to the invention, the second material comprises calcium sulphate (hemi-hydrate and/or di-hydrate) in any crystal configuration. Calcium sulphate may be abbreviated or denoted as "CaS" in present text.

Such materials, referred herein as a "second material", are usually absorbed by the body within a few days, week or months, such as e.g. about 1 day, such as e.g. about 2 days, such as e.g. about 3 days, such as e.g. about 4 days, such as e.g. about 5 days, such as e.g. about 6 days, such as e.g. about 7 days, such as e.g. about 8 days, such as e.g. about 9 days, such as e.g. about 10 days, such as e.g. about 11 days, such as e.g. about 12 days, such as e.g. about 13 days, such as e.g. about 2 weeks, such as e.g. about 3 weeks, such as e.g. about 4 weeks, such as e.g. about 5 weeks, such as e.g. about 6 weeks, such as e.g. about 7 weeks, such as e.g. about 2 months, such as e.g. about 3 months, or such as e.g. about 4 months etc.

In one aspect, the second material is usually absorbed by the body of the subject within e.g. about 1 day, such as e.g. about 2 days, such as e.g. about 3 days, such as e.g. about 4 days, such as e.g. about 5 days, such as e.g. about 6 days, such as e.g. about 7 days, such as e.g. about 8 days, such as e.g. about 9 days, such as e.g. about 10 days, such as e.g. about 11 days, such as e.g. about 12 days, such as e.g. about 13 days, such as e.g. about 2 weeks, such as e.g. about 3 weeks, such as e.g. about 4 weeks, such as e.g. about 5 weeks, such as e.g. about 6 weeks after administration to the subject.

In a further aspect, the second material is usually absorbed by the body of the subject within e.g. about 1 day to about 6 weeks after administration to the subject. In one aspect, the second material is usually absorbed by the body of the subject within about 6 weeks after administration to the subject.

In yet a further aspect, the first material is absorbed very slowly by the organism, such as e.g. in the range of weeks, months and even years, such as e.g. about 2 weeks, such as e.g. about 3 weeks, such as e.g. about 4 weeks, such as e.g. about 5 weeks, such as e.g. about 6 weeks, such as e.g. about 7 weeks, such as e.g. about 2 months, such as e.g. about 3 months, such as e.g. about 4 months, such as e.g. about 6 months, such as e.g. about 8 months, such as e.g. about 10 months, such as e.g. about 12 months, such as e.g. about 18 months, such as e.g. about 2 years, or such as e.g. several years etc., and wherein the second material is usually absorbed by the body within a few days, week or months, such as e.g. about 1 day, such as e.g. about 2 days, such as e.g. about 3 days, such as e.g. about 4 days, such as e.g. about 5 days, such as e.g. about 6 days, such as e.g. about 7 days, such as e.g. about 8 days, such as e.g. about 9 days, such as e.g. about 10 days, such as e.g. about 11 days, such as e.g. about 12 days, such as e.g. about 13 days, such as e.g. about 2 weeks, such as e.g. about 3 weeks, such as e.g. about 4 weeks, such as e.g. about 5 weeks, such as e.g. about 6 weeks, such as e.g. about 7 weeks, such as e.g. about 2 months, such as e.g. about 3 months, or such as e.g. about 4 months etc.

In a further aspect, the first material is absorbed very slowly by the organism, such as e.g. about 6 months, such as e.g. about 8 months, such as e.g. about 10 months, such as e.g. about 12 months, such as e.g. about 18 months, such as e.g. about 2 years, or between about 6 months to about 2 years after administration to the subject, and wherein the second material is usually absorbed by the body of the subject within e.g. about 1 day, such as e.g. about 2 days, such as e.g. about 3 days, such as e.g. about 4 days, such as e.g. about 5 days, such as e.g. about 6 days, such as e.g. about 7 days, such as e.g. about 8 days, such as e.g. about 9 days, such as e.g. about 10 days, such as e.g. about 11 days, such as e.g. about 12 days, such as e.g. about 13 days, such as e.g. about 2 weeks, such as e.g. about 3 weeks, such as e.g. about 4 weeks, such as e.g. about 5 weeks, such as e.g. about 6 weeks, or within e.g. about 1 day to about 6 weeks after administration to the subject.

As indicated above, the first material may be dispersed in the second material, such as e.g. HA (hydroxyapatite) being dispersed or mixed in a paste of calcium sulphate (CaS), and may be regarded as embedded in CaS. Solid formulations may be in any form such as e.g. tablets, pellets, beads, sticks etc. of any suitable size such as allowing implantation into the body of the subject or injection into the body of the subject. With respect to liquid or semi-solid compositions, these may be administered to the subject by means of injection or any other suitable means. The composition for use according to the invention comprising the first and a second material may be such that once administered to the subject, the composition cures into a solid at the site of administration. The administration of the composition may suitably be at the site of the body in need of treatment such as e.g. into or near bone tissue either for treatment of an ongoing bacterial infection or for prophylactic treatment or preventive treatment of bacterial infections.

In one aspect, the first material may be hydroxyapatite (HA) in any form and the second material, calcium sulphate (CaS) is in any form or crystal configuration. In a further aspect, the particulate first material (HA) may be embedded into the second material (CaS).

As is apparent from the description, the first and second material may act as carriers for the one or more pharmaceutical compounds and are from a pharmacological perspective inert.

Specifically, the pharmaceutically active compound, which may be one or more pharmaceutically active compounds, is a compound capable of treating a bacterial infection.

As mentioned herein, the composition for use according to the invention may be administered into soft tissue or may be bone tissue.

In the context of present invention, the compositions for use as disclosed herein is for use in the treatment of bacterial infections of any kind. In a specific embodiment, present invention relates to treating bacterial infections in bone tissue.

In a further specific embodiment, present invention relates to treatment of deep bone infections (DBI's).

Once the material or composition according to the invention has been administered to the subject, the second material is absorbed by the body or erodes in the body of the subject so as to expose the finely particulate structure or the first material having a very high effective surface area. The period during which the absorption or erosion of the second material takes place varies with the material used as second material. Thus the period in questions may be at least a few hours , or within e.g. about 1 day, such as e.g. about 2 days, such as e.g. about 3 days, such as e.g. about 4 days, such as e.g. about 5 days, such as e.g. about 6 days, such as e.g. about 7 days, such as e.g. about 8 days, such as e.g. about 9 days, such as e.g. about 10 days, such as e.g. about 11 days, such as e.g. about 12 days, such as e.g. about 13 days, such as e.g. about 2 weeks, such as e.g. about 3 weeks, such as e.g. about 4 weeks, such as e.g. about 5 weeks, such as e.g. about 6 weeks, or within e.g. about 1 day to about 6 weeks after administration to the subject. After the absorption period of the second material, one or more further pharmaceutical compositions may be optionally and additionally administered to the subject. Consequently, one or more pharmaceutical compositions may be administered after at least a few hours , or within e.g. about 1 day, such as e.g. about 2 days, such as e.g. about 3 days, such as e.g. about 4 days, such as e.g. about 5 days, such as e.g. about 6 days, such as e.g. about 7 days, such as e.g. about 8 days, such as e.g. about 9 days, such as e.g. about 10 days, such as e.g. about 11 days, such as e.g. about 12 days, such as e.g. about 13 days, such as e.g. about 2 weeks, such as e.g. about 3 weeks, such as e.g. about 4 weeks, such as e.g. about 5 weeks, such as e.g. about 6 weeks, or within e.g. about 1 day to about 6 weeks after the first and/or second material has been administered to the subject.

Alternatively, the one or more pharmaceutical compositions are administered e.g. about 6 weeks to about 1 year, about 18 months, or about 2 years after administration of the composition according to the invention.

The one or more pharmaceutical compositions may be administered one or more times, such as e.g. 2 or more time, 3 or more times, 4 or more times, 5 or more times, 6 or more times, 7 or more times, 8 or more times, 9 or more times, 10 or more times etc.

As is implied in present description, the composition according to the invention may be an extended release composition, wherein the one or more pharmaceutical compounds are released from the first and the second material over an extended period of time. In a particular aspect, the composition is administered to a particular tissue, whereby the one or more pharmaceutical compounds are released from the first and the second material over an extended period of time at or near the site of administration. This in turn may result in not having to administer the same or different pharmaceutical compounds at any later time.

The composition according to the invention enables the one or more pharmaceutical compounds to be released from the first and optionally second material for an extended period of time such as e.g. at least 1 day, at least about 3 days, at least about 7 days, at least about 14 days, at least about 21 days, at least about 28 days, or at least 35 days or more.

The one or more pharmaceutical compositions comprise at least two or more different active ingredients, such as 3 or more, 4 or more, or 5 or more etc.

Such active ingredients may display different affinities toward the first and the second material.

In a one aspect, the first and the second material may be pre-soaked with one or more pharmaceutical compounds. Alternatively, the first and the second material, may be mixed together with one or more pharmaceutical compounds. The pre-soaking or mixing may take place prior to any administration to the subject.

In another aspect, the pharmaceutical compound may be administered to the subject after administration of the first and the second material.

In yet a further aspect, the pharmaceutical compound may be pre-soaked or mixed with the first and the second material, and may optionally also be administered one or more times after administration of the pre-soaked or mixed first and optionally the second material.

The pre-soaking or mixing may be such that one type of one or more pharmaceutical compounds is/are pre-soaked into or mixed with a first material, and the same or a different type of one or more pharmaceutical compounds is/are pre-soaked into or mixed with a second material.

The one or more pharmaceutical compounds according to the invention may in principle be any pharmaceutical compound that shows an affinity for the first material used in the composition according to the invention. In one aspect, the pharmaceutical compound may be any pharmaceutical compound showing an affinity for hydroxyapatite particles.

Thus, present invention also relates to a composition for use, wherein the one or more administered drugs display different affinities towards the first and the second material. Thus in one aspect, present invention relates to a bi-phasic composition, various different pharmaceutical compounds may be employed such that e.g. one pharmaceutical compound shows high affinity towards the particulate first material (and low affinity towards the second material), and another pharmaceutical compound shows high affinity towards the second material (and low affinity towards the first material) etc.

In one aspect, the second material may be mixed or pre-loaded with a pharmaceutical compound which optionally shows high affinity for the second material, and then further mixed with the first material. Also, alternatively, the first material may be mixed or exposed to a pharmaceutical compound displaying high affinity to the first material. The above mentioned first and second materials may be mixed and administered to the subject in need.

In one aspect, present invention also relates to a composition for use comprising a first particulate material, wherein the material is present in nano-sized particles. The material may or may not be pre-loaded with a pharmaceutical compound. Such particles may be administered as a suspension or otherwise dispersed in any suitable solvent such as e.g. as saline solution, which is suitably a physiological saline solution. Such composition may be used for various applications such as e.g. administration into or near an infected tissue, where the particles will penetrate the bacterial cells to exert their action.

In one aspect, the pharmaceutical compound displaying a high affinity towards the first material is rifampicin.

Also, present invention relates to one or more pharmaceutical compound displaying a low or no affinity towards the first material.

In one aspect, the pharmaceutical compound displaying a low or no affinity towards the first material is gentamycin.

In yet a further aspect, the pharmaceutical compound displaying a low or no affinity is vancomycin.

In a further aspect, the pharmaceutical compounds is a combination of vancomycin and rifampicin.

Alternatively, the pharmaceutical compounds is a combination of gentamicin and rifampicin.

As yet a further alternative, the pharmaceutical compounds may be a combination of gentamicin, vancomycin and rifampicin.

As is evident from the above, present invention relates to a composition for use in treating, or preventing, or reducing or altogether eliminating the emergence of bacterial resistance towards any antibiotic compound. Also, in a particular aspect, present invention relates to a composition for use in treating, or preventing, or reducing or altogether eliminating the emergence of bacterial resistance towards any antibiotic compound, such as e.g. bacterial strains exhibiting rifampicin resistance.

In another aspect, present invention relates to the composition and pharmaceutical compounds as disclosed herein for use in treatment in bacterial infections in any type of tissue, such as e.g. in bone tissue and in particular deep bone infections (DBIs).

Present invention also enables prevention, or prolongs the period for development of bacterial resistance towards the administered pharmaceutical compounds.

Present invention also enables an effective treatment of, or reduction of, or elimination of, or prevention of planktonic bacteria.

With respect to administration of the pharmaceutically active compound, it is to be understood that any conventional method of administration may be employed which has been previously approved by clinical authorities. If for example a drug has been approved for oral administration, the same administration rout may be employed according to the invention. Thus, the administration is usually results in the pharmaceutically active compound entering the systemic circulation of the subject. As previously mentioned, the administration may be e.g. oral or intravenous.

In another aspect of the invention, the pharmaceutically active compound may be administered near or into e.g. an implant.

In one aspect, the administration may be by injection. In this respect, the injection may be in form of a paste comprising the first and optionally the second material which may have been further premixed or soaked with one or more pharmaceutical compounds, wherein the paste is allowed to cure in the tissue. Alternatively, the curing may take place prior to administering of the composition as a whole to the subject. As mentioned herein, the one or more pharmaceutical compounds may be further administered after administration of the first and optionally the second material (which may or may not have been premixed or soaked with one or more pharmaceutical compounds).

As mentioned herein the first material used according to the invention has to be in a finely divided solid state, such as e.g. in small particles so as to provide for a high effective surface. The first material may be such that 1 ml of the material contains at least 0.5 million particles, such as e.g. at least 1 million particles, such as e.g. at least 2 million particles, such as e.g. at least 3 million particles, such as e.g. at least 4 million particles, such as e.g. at least 5 million particles, or such as e.g. at least 10 million particles. In one aspect, the first material according to the invention is apatite or hydroxyl apatite.

The particles according to the invention may be in range of micro-particles or nanoparticles. The particle size is preferably e.g. less than 200 $\mu$m, such as less than 100 $\mu$m, less than 50 $\mu$m, less than 35 $\mu$m, less than 20 $\mu$m or less than 10 $\mu$m. Moreover, the particles may be between about 0.1 and about 50 $\mu$m. In another aspect the microparticles may be in range of about 1 $\mu$m to about 500 $\mu$m, such as .e.g about 1 $\mu$m to about 100 $\mu$m, about 1 $\mu$m to about 50 $\mu$m, about 1 $\mu$m to about 25 $\mu$m, about 1 $\mu$m to about 15 $\mu$m, about 1 $\mu$m to about 10 $\mu$m, or about 1 $\mu$m, about 5 $\mu$m, about 10 $\mu$m, about 15 $\mu$m, about 20 $\mu$m, about 25 $\mu$m, about 30 $\mu$m, about 35 $\mu$m, about 40 $\mu$m, about 45 $\mu$m, about 50 $\mu$m, about 75 $\mu$m, about 100 $\mu$m, about 500 $\mu$m etc.

In one aspect, the particles of the first material is in range of e.g. about 0,5 $\mu$m to about 50 $\mu$m.

In one aspect, the particles of the first material is in range of e.g. about 1 $\mu$m to about 25 $\mu$m.

In one aspect, the particles of the first material is in range of e.g. about 1 $\mu$m to about 10 $\mu$m.

In a preferred embodiment, the drug may be administered once and once only and the particles in the first material are micro sized particles. As mentioned herein, the first material is hydroxyapatite (HA) in any configuration.

As mentioned herein, the administered drug or drugs may be administered several times at different occasions as deemed necessary by the clinicians.

In yet a further aspect, the particle size may be in range of e.g. about 1 nm to about 200 nanometres (nm), such as e.g. less than about 150 nm, less than about 100 nm, less than about 50 nm, less than about 45 nm, less than about 40 nm, less than 35 nm, less than about 30 nm, less than about 25 nm, less than about 20 nm, less than about 15 nm, or less than about 10 nm. In one aspect the particle size is in range of e.g. about 1 nm to about 100 nm. In a further aspect, the particles of the first material may be about 1 nm, such as e.g. 5 nm, such as e.g. about 10 nm, such as e.g. about 15 nm, such as e.g. about 20 nm, such as e.g. about 25 nm, such as e.g. about 30 nm, such as e.g. about 35 nm, such as e.g. about 40 nm, such as e.g. about 45 nm, such as e.g. about 50 nm, such as e.g. about 100 nm, such as e.g. about 150 nm, or such as e.g. about 200 nm.

In one aspect, the particle size may be in range of about 40 nm to about 100 nm.

In a further aspect, the particle size may be in range of 10 nm to about 50 nm.

It has been surprisingly discovered by the inventors of present invention that in such instances that multiple administration of a drug or drugs (pharmaceutical compounds) is deemed necessary, the affinity towards nano-sized particles in the first material is higher for nano-sized particles in the sense that upon additional administration (i.e. repeated administration) of one or more pharmaceutical compositions, nano-sized particles of the first material displays a higher affinity than larger micro-sized particles. Thus, repeated administration of one or more pharmaceutical compounds may be employed as a means to re-load the nano-sized particles in the first material.

This also has a benefit, in that nano-sized particles has the ability to enter a cell, such as e.g. a bacterial cell, and thus the local concentration of a pharmaceutical compound may be higher than in the systemic circulation and may thus attain concentrations equal to or above clinically relevant amounts to enable successful treatment of e.g. a bacterial infection.

In another aspect, the first material according to the invention may be a composition comprising a mixture or micro-sized particles and nano-sized particles. Thus the first material may comprise at least partly particles in the range of about 1 μm to about 500 μm, such as .e.g about 1 μm to about 100 μm, about 1 μm to about 50 μm, about 1 μm to about 25 μm, about 1 μm to about 15 μm, about 1 μm to about 10 μm, or about 1 μm, about 5 μm, about 10 μm, about 15 μm, about 20 μm, about 25 μm, about 30 μm, about 35 μm, about 40 μm, about 45 μm, about 50 μm, about 75 μm, about 100 μm, about 500 μm etc, and/or particles in the range of e.g. 1 and 200 nanometres (nm), such as e.g. less than 100 nm, less than 50 nm, less than 35 nm, less than 20 nm or less than 10 nm. In one aspect the particle size is in range of e.g. about 100 nm to about 1 nm.

Thus, in one aspect, the composition according to the invention may comprise only a first material as disclosed herein. As mentioned herein the first material is hydroxyapatite (HA). The first material may be a mixture of micro-sized particles or nano-sized particles as mentioned herein, or alternatively may be a mixture of micro-sized particles and nano-sized particles, and thus a combination of micro-sized particles and nano-sized particles. For example, micro-sized particles may be in range of about 1 μm to about 500 μm, or about 1 μm to about 10 μm, and the nano-sized particles may be in range of about 1 nm to about 200 nm, or about 40 nm to about 100 nm.

In another aspect, present invention relates to a composition comprising first and a second material as disclosed herein. The first material may be a mixture of micro-sized particles and/or nano-sized particles as mentioned herein. In one example, the first material may be a mixture of micro-sized particles and nano-sized particles as mentioned herein, and consequently may be a mixture of micro-sized particles or nano-sized particles, or a combination of micro-sized particles and nano-sized particles. For example, micro-sized particles may be in range of about 1 μm to about 500 μm, or about 1 μm to about 10 μm, and wherein the nano-sized particles may be in range of about 1 nm to about 200 nm, or about 40 nm to about 100 nm, or about 10 nm to about 50 nm.

In a particular aspect, the composition may comprise a first material (HA) and a second material (CaS), wherein the first material has a particle size of about 10 μm and/or 1 nm to about 50 nm, or about 50 nm. As mentioned herein, the first material may be embedded or dispersed in the second material.

Various ratios and relations between the amount of micro-sized and nano-sized particles may be present in a composition according to the invention. For example, the relation between the various partciles sizes may be in range of about 1% (wt%) to about 99.9% (wt%) between nano:micro-sized particles, such as e.g. about 10% to about 90%, about 15% to about 85%, about 20% to about 80%, about 25% to about 75%, about 30% to about 70%, about 35% to about 65%, about 40% to about 60%, about 45% to about 55%, or about 50% to about 50%, about 55% to about 45% , about 60% to about 40%, about 65% to about 35%, about 70% to about 30%, about 75% to about 25%, about 80% to about 20%, about 85% to about 15%, about 90% to about 10%, about 95% to about 5%, or about 99,9% to about 1%. In one aspect, the ratio or relationship is about 50% to about 50% between nano:micro-sized particles. In one further aspect, the invention relates to a composition according to the invention wherein about 33,3% are micro-sized particles, 33,3% are nano-sized particles, and the remaining about 33,3% may be midsized particles which are not overlapping with the particle size intervals of the micro-sized and nano-sized particles.

In another aspect, the inventors of present invention have found that a certain amount of the first material is capable of binding a certain amount of the administered drug or drugs. In one non-limiting example, the first material may be hydroxyapatite. The amount of the first material may be e.g. at least about 1 mg, such as e.g. at least about 5 mg, such as e.g. at least about 10 mg, such as e.g. at least about 15 mg, such as e.g. at least about 20 mg, such as e.g. at least about 25 mg, such as e.g. at least about 30 mg, such as e.g. at least about 35 mg, such as e.g. at least about 40 mg, such as e.g. at least about 45 mg, such as e.g. at least about 50 mg, or at least about 500mg . Alternatively, the amount of the first material may be in range of about 1 mg to about 50 mg, such as e.g. 5 mg to about 40 mg, such as e.g. about 5 mg to about 35 mg, such as e.g. about 5 mg to about 30 mg, such as e.g. about 5 mg to about 25 mg, such as e.g. about 5 mg to about 20 mg, such as e.g. about 5 mg to about 15 mg, such as e.g. about 5 mg to about 10 mg. As a further alternative, the amount of the first material in a composition according to present invention may be about 5 mg, such as e.g. about 10 mg, such as e.g. about 15 mg, such as e.g. about 20 mg, such as e.g. about 25 mg, such as e.g. about 30 mg, such as e.g. about 35 mg, such as e.g. about 40 mg, such as e.g. about 45 mg, such as e.g. about 50 mg.

It has been found that micro-sized as well as nano-sized particles of the first material, which in one aspect may be hydroxyapatite (HA), in the amount of about 5 mg is capable of binding about 10% of the administered drug. It has further been found that 10 mg of the first material is capable of binding about 30% of the administered drug and that 20 mg of the first material is capable of binding about 75% of the administered drug. In another aspect, it has been found that about 30mg to about 40 mg is capable of binding nearly 100% of the administered drug. Thus, in one aspect the amount of the first material is preferably in range of about 30 mg to about 40 mg. The amount of the first material is to be understood as the amount being optionally mixed with the second or even third material that may be in other ranges of amounts, in a composition according to the invention.

The ratio between the first and the second material may be in range of about about 1% to about 99.9% between first material:second material, such as e.g. about 10% to about 90%, about 15% to about 85%, about 20% to about 80%, about 25% to about 75%, about 30% to about 70% , about 35% to about 65%, about 40% to about 60%, about 45% to about 55%, or about 50% to about 50%, about 55% to about 45% , about 60% to about 40%, about 65% to about 35%, about 70% to about 30%, about 75% to about 25%, about 80% to about 20%, about 85% to about 15%, about 90% to about 10%, about 95% to about 5%, or about 99,9% to about 1 %. In one aspect, the composition comprises only a first material. In one aspect, the ratio or relationship is about 40% to about 60% between first material:second material. In one non-limiting example, the first material may be e.g. HA (hydroxyapatite) in an amount of 40% (wt%) and the second material may be e.g. CaS (calcium sulphate) in an amount of 60% (wt%).

In one aspect, the first material such as e.g. hydroxyapatite is provided as hydroxyapatite particles (e.g. sintered hydroxyapatite particles) to be mixed with the second material, i.e. calcium sulphate powder and water for the calcium sulphate to set, whereby the calcium phosphate particles becomes embedding in the calcium sulphate phase after setting. The water may be seen as a non-limiting example of a third material. A third material may be any additional solvent or further solid substance acting as diluent or mixing agent to the first and optionally the second material.

In one aspect, the composition according to the invention comprises a first and a second material, wherein the first material may be administered as a particulate material which may optionally be in form of an emulsion, dispersion, solution etc. In such case, optionally a further diluent is added which acts as a carrier to the first material.

In another aspect, the composition according to the invention comprises a first and a second material. The composition may then be prepared by conventional mixing known in the art, which may further comprise addition of a solvent or other diluent. In one example, the mixing of the components of the composition may take place at normal room temperature and/or under normal atmospheric pressure. Alternatively, the mixing may take place under vacuum or reduced atmospheric pressure. It has been observed that the wettability affecting chemical binding of the finished composition is increased by mixing under vacuum or reduced atmospheric pressure.

In one aspect, the invention relates to a method of loading one or more pharmaceutical compounds onto the first and/or second material of present invention. Such method is outlined in accordance with claim 11.

The one or more pharmaceutical compounds may be same or different and may have different affinities for the first and second material. The one or more pharmaceutical compounds may be dissolved in any suitable solvent such as a physiological saline solution, water or other aqueous solution, buffered solution, or any suitable organic solvent or any mixtures thereof.

The composition according to the invention may be e.g. in form of a pellet. The pellet may be prepared by mixing the first material with the second material (mixing Ha with CaS) in a powder form, in a suitable solvent such as e.g. a saline aqueous solution (0,9 wt% NaCl in water; known as normal saline), and/or hyaluronic acid, and/or iodine-based mixing solution. In one aspect, the resulting slurry is supplied with vancomycin. The slurry may be employed as a liquid or semi-solid composition and may be injected into the subject. Alternatively, the slurry may be allowed to cure into a solid composition and may be implanted into the subject.

In one aspect, the resulting slurry above is supplied with gentamicin. The slurry may be employed as a liquid or semi-solid composition and may be injected into the subject. Alternatively, the slurry may be allowed to cure into a solid composition and may be implanted into the subject.

In one aspect, the resulting slurry above is supplied with rifampicin. The slurry may be employed as a liquid or semi-solid composition and may be injected into the subject. Alternatively, the slurry may be allowed to cure into a solid composition and may be implanted into the subject.

For example, the composition may comprise e.g. vancomycin or gentamicin, wherein the composition is administered to a subject in need. Thereafter, an additional pharmaceutical compound is administered to the subject. The additional pharmaceutical compound may be administered locally near the implanted or administered composition or systemically to the subject. In one aspect, the additional pharmaceutical compound is rifampicin.

The period during which the one or more pharmaceutical compounds is contacted with the particulate first material and/or a second material, may be within range of minutes, hours or days, or 1 hour, such as e.g. about 3 hours, such as e.g. about 6 hours, such as e.g. about 12 hours, such as e.g. about 24 hours, or 48 hours.

After contacting/incubating the one or more pharmaceutical compounds with the particulate first material and/or a second material, the resulting mixture may be separated from the solution or suspension comprising the one or more pharmaceutical compounds by centrifugation or filtration etc. the resulting particulate material may be rinsed with any suitable solvent and optionally thereafter allowed to dry.

As is apparent form present invention, the composition according to the invention may be regarded as an extended release composition, such that the two or more pharmaceutical compounds are released from the first and second material, are continuously released from the said first and second material in a prolonged manner or over an extended period of time. Such an extended period of time may be e at least 1 day, e.g. at least 2 days, e.g. at least 3 days, e.g. at least 5 days, e.g. at least 7 days, e.g. at least 14 days, e.g. at least 21 days, e.g. at least 28 days, or at least 35 days, after administration of the composition according to the invention.

Thus according to the invention, it may be sufficient to provide the subject, i.e. administer the composition once during a period of 1 day, 2 days, 3 days, 5 days, 7 days, 14 days, 21 days, 28 days, or 35 days.

Consequently, present invention provides a composition allowing for a continuous release of the antibiotic compounds present in the composition.

In one aspect, the composition may comprise about 50 mg to about 5000 mg of HA/CaS.

In a non-limiting example, the composition may comprise about 100 mg to about 2000 mg, or such as e.g. about 300 mg to about 1000 mg, or about 500 mg of HA/CaS.

In a further aspect, the composition may comprise one or more pharmaceutical compounds in a clinically relevant amount. In a particular aspect, such clinically relevant amount may be in any range of from about 0.5 mg to about 2000 mg. In a non-limiting aspect, the range/amount of e.g vancomycin may be in range of about 1 mg to about 50 mg, such as e.g. about 25 mg.

In one aspect, gentamicin may be present in an amount of about 1 mg to about 50 mg, such as e.g. about 25 mg.

In yet a further aspect, rifampicin may be present in an amount of about 0.1 mg to about 20 mg, such as e.g. about 8 mg.

Importantly, the inventors of present invention have surprisingly found that even after a prolonged treatment employing the composition according to present invention, no bacterial resistance arises. One non-limiting examples is seen in *e.g.* rifamipicin resistant bacteria, where no build-up of antibiotic resistance is seen even after 35 days after administration of the composition according to the invention.

The invention is further illustrated in the following examples.

## Examples

## Materials and methods

### *Materials used*

Medical grade rifampicin (RIF) (Rifampicin Ebb, 600 mg; Sanofi S.p.A, Anagni, Italy) was purchased from the local pharmacy (Apoteket AB, Sweden). CaS/HA composite containing GEN (gentamicin) (Cerament® G) or VAN (Vancomycin) (Cerament® V) were supplied by Bonesupport AB, Lund, Sweden. The particle size of the hydroxyapatite particles in the Cerament compositions are in range of about 1 $\mu$m to about 10 $\mu$m. Phosphate buffered saline (PBS, pH 7.0-7.2) was supplied by Cytiva, Utah, US. Sodium pyruvate were purchased from Thermo scientific, U.S.A. Heat inactivated fetal bovine serum (FBS), trypsin-EDTA solution (0.25%), sodium pyruvate solution, and 3-(4,5-Dimethylthiazol-2-yl)-2,5-diphenyltetrazolium bromide (MTT) reagent were purchased from Sigma Aldrich, Germany. MC3T3-E1 subclone 4 cells were purchased from ATCC (VI, U.S.A). $\alpha$ -minimum essential medium ($\alpha$MEM) without phenol red was purchased from Life Technologies Europe BV (Stockholm, Sweden).

### *Preparation and characterization of antibiotic loaded CaS/HA composites*

*Antibiotic loaded CaS/HA composite synthesis:* The following combinations of CaS/HA and antibiotics were prepared: CaS/HA-VAN, CaS/HA-VAN+RIF, CaS/HA-GEN, CaS/HA- GEN+RIF, and CaS/HA-RIF. For CaS/HA-VAN, CaS/HA-VAN+RIF composites, 500 mg of CaS/HA powder (Cerament V, Bonesupport AB, Sweden) was hand-mixed in a 48-well plate with either only VAN (24.57 mg) or in combination of RIF (8.11 mg) dissolved in 215 $\mu$L mixing-solution (C-TRU, Bonesupport AB, Sweden) using a sterile wooden stick for 30 seconds at 22 $\pm$ 1°C both in clockwise and anticlockwise directions after every 3 rounds. The paste was transferred to a 1 mL syringe (B BRAUN, Melsungen, Germany) connected to an 18G needle (Ø =1.2 mm, L=4 cm) and extruded into a mould with hemispherical wells (Ø=4.8 mm) to obtain uniform sized pellets for evaluating antibiotic release profile, material degradation and continuous Kirby-Bauer disk diffusion assay. CaS/HA-GEN or CaS/HA-RIF composites were prepared by mixing 500 mg of CaS/HA powder (Cerament G, Bonesupport AB, Sweden) and GEN (24.57 mg) or RIF (8.11 mg) dissolved in 283.5 $\mu$L mixing-solution (Bonesupport AB, Sweden). For CaS/HA-GEN+RIF composites, preparations were the same but with the difference of intermittent mixing technique. Briefly, 500 mg of CaS/HA powder (Cerament G, Bonesupport AB, Sweden) was first hand-mixed with GEN (24.57 mg) dissolved in 200 $\mu$L mixing-solution (Bonesupport AB, Sweden) and 30 seconds later, RIF (8.11 mg) dissolved in 83.5 $\mu$L mixing-solution (Bonesupport AB, Sweden) added and mixed again. This intermittent mixing technique was adopted to prevent the rapid setting of the composite when GEN and RIF mixed simultaneously. CaS/HA-GEN, CaS/HA-RIF and CaS/HA-GEN+RIF hemispherical pellets were prepared as mentioned previously.

*Injectability and setting time*

CaS/HA composites were prepared as mentioned above. The injectability of the composite was then evaluated by extruding 250 μL of the paste manually through a graduated 1 mL syringe connected to an 18G needle (Ø =1.2 mm, L=4 cm) into a mould with hemispherical wells (Ø=4.8 mm). Total weight of the syringe with paste before and after extrusion were measured. To normalize the amount of composite that remained in the needle and syringe tip after injection, the mean of the paste extruded from the syringe at the first time point (for GEN, 4 min; VAN and RIF, 3 min) was calculated for the tested concentrations (normalized value). Injectability (%) was calculated by dividing the weight of the paste extruded from the syringe with a normalized value. Each test was repeated for three times at various time points (GEN: 4, 6 and 8 min; VAN and RIF: 3, 5, and 7 min). The composite extruded into the mould wells was left to solidify as per manufactures recommendations for 20 min and to confirm the setting of the tested composites, the consistency of the pellets were evaluated manually.

$$\text{Injectability (\%)} = \frac{\text{weight of the paste extruded from the syringe}}{\text{normalized value}} \times 100$$

*Antibiotic release profile*

For each combination, pellets in triplets were placed in 2 mL microcentrifuge tube containing 1 mL of sterile PBS and placed at 37°C. At different time points (Day 1, 3, 7, 14, 21, 28 and 35) PBS was collected and tubes were supplemented with fresh 1 mL PBS. The amount of VAN and RIF released from the pellets were quantified using liquid chromatography-tandem mass spectrometry (LC-MS/MS) method (Thermo Fisher Scientific, USA) and GEN quantification was done by cloned enzyme donor immunoassay (CEDIA) on Roche Cobas, C501 (Roche Diagnostics, Risch-Rotkreuz, Switzerland).

*Testing of Antibacterial effects*

**Bacterial Strains and culture conditions**

*For in-vitro* bacterial experiments, *Staphylococcus aureus* standard strain oobtained from American Type Culture Collection (*Staphylococcus aureus* ATCC® 25923™), and *S. aureus* clinical strain P3, isolated from a case of prosthetic joint infection at Lund University Hospital were used. The strains were preserved in -80 °C and before experiments subcultured onto in-house made sheep blood agar plates. Prior to use, a minimum of two subcultures were done.

*Efficacy on planktonic bacteria by continuous Kirby-Bauer disk diffusion assay*

The antimicrobial efficacy of composites on planktonic bacteria were tested by Kirby-Bauer disk diffusion assay. For tested combinations of CaS/HA-antibiotics, pellets in triplets were used for each strain of *S. aureus.* The bacteria were inoculated into sheep blood agar plates and incubated at 37°C for 24 h. Then, bacterial suspensions were prepared by dissolving the bacteria in sterile saline (NaCl 0,9%) to an optical density (O.D.) 600 = 0,1 ± 0,005 as measured by spectrophotometer (Thermo Scientific GENESYS 20). By lawn culture, bacteria were inoculated on in-house made Mueller-Hinton agar (MHA) plates and using sterile forceps, pellets were transferred to the inoculated plates within 3-15 min of inoculation. The plates were then incubated at 37 °C for 24 h, and diameter of the zone of inhibition (ZOI) was measured using a standard ruler. After measuring the ZOI, the pellets were transferred to new set of plates inoculated with bacteria using sterile forceps, and the procedure repeated until their zone of inhibition became zero, or for a maximum of 28 days. At predetermined time points (day 1, 3, 7, 14, 21, and 28) MHA plates exhibiting ZOI were imaged using ChemiDoc™ MP imaging system (Bio-Rad Laboratories, Hercules, CA). For GEN and RIF pellets, using CLSI guidelines, ZOI of ≥15 and ≥20 mm respectively taken as strong antibacterial effect, and an ZOI of less than these were represented moderate antibacterial effect. For VAN, ZOI of ≥17 mm used as surrogate for strong antibacterial effect.

**Antibacterial effect of pellets after day-35:** To mimic the *in vivo* conditions, after day-35, the antibacterial of the pellets that have been used for antibiotic release assay were tested in a similar way as mentioned above. Briefly, following the day-35 fraction collection for antibiotic release assay, the pellets were crushed to form a paste, and using a sterile spatula, it was transferred to the MHA plates inoculated with *S. aureus* ATCC 25923. After 18 h of incubation, the ZOI (Zone Of Inhibition) were noted and plates were imaged using ChemiDoc™ MP imaging system (Bio-Rad Laboratories, Hercules, CA).

*Efficacy on preformed biofilms*: Using a modified biofilm quatification method, the antibiotics released at different time points (Day 1, 3, 7, 14, 21, 28 and 35) from the pellets of CaS/HA-antibiotic composites were tested for their ability to distrupt preformed biofilm. S. *aureus* were grown in tryptic soy broth supplimented with 1.0% glucose (TSBG) in a rotary shaker (180 rpm) at 37 °C for 16-18 h. Then, the Bacterial suspension were centrifuged at 4000 rpm for 10 min and immediately resuspended in fresh TSBG. The resuspended bacterial suspension were diluted to optical density 0.17 measured at 600 nm (Thermo Scientific GENESYS 20). From the diluted suspension, 11.4 $\mu$L were added to 138.6 $\mu$L of TSBG in the wells of 96-well flat bottom tissue culture treated polystyrene microtitre plate (Costar, Kennebunk, ME, USA) coated with poly-L-lysine (0.2 mg/mL) (Sigma-Aldrich, Germany) and incubated at 37 °C statically. Biofilm were allowed to form on the wells for 48 h with change of media after 24 h of incubation. After incubation, biofilms were gently washed three times with sterile PBS to remove the planktonic bacteria. Thereafter, 200 $\mu$L of TSBG and TSBG-antibiotic fractions mixture (1:1) were added to the control and test wells respectively, and incubated at 37 °C for 24 h. The wells were then gently washed two times with PBS and fixed with 150 $\mu$L of methanol for 20 min. After air drying for overnight, the remaining biofilms were stained with 150 $\mu$L 1% (w/v) crystal violet solution (Sigma-Aldrich, Germany) for 15 min at room temperature. The wells were washed three times with PBS and air dried before adding 95% ethanol to resolubilize the dye bound to cells. After 30 min, 100 $\mu$L from each well was transferred to new 96-well plate and optical density was measured at 590 nm using a microplate reader (iMark™, Bio-Rad, Japan). The abosrbance from the test wells which correlates to the amount of biofilm remained after exposure to the antibiotic fractions, were comapred with the absorbance of untreated control wells. The efficacy of the antibiotic fractions were tested on both *S. aureus* ATCC 25923, and *S. aureus* clinical strain P-3. For each tested group of antibiotic fractions, five separate wells were used (n=5).

**Scanning Electron Microscopy of Biofilms**

As mentioned in above, biofilms were first allowed to form on glass coverslips (Ø=13 mm) coated with poly-L-lysine (0.2 mg/mL) for 48 h in a 12-well plate (Costar, Kennebunk, ME, USA) with change of media after 24 h of incubation. After incubation, biofilms were gently washed three times with sterile PBS to remove the planktonic bacteria. Thereafter, 700 $\mu$L of TSBG and TSBG-antibiotic fractions mixture (1:1) were added to the control and test wells respectively, and incubated at 37 °C for 24 h. The wells were then gently washed two times with PBS and fixed in 2.5% glutaraldehyde in 0.15 M sodium cacodylate buffer (pH 7.4) for overnight at room temperature. Fixed samples were washed with 0.15 M sodium cacodylate (pH 7.4) for 10 min to a minimum of four cycles, and dehydrated with increasing concentrations of ethanol followed by critical point drying with liquid carbon dioxide in Baltec CPD030, using absolute ethanol as the intermediate solvent. Samples were then mounted onto aluminium holders and sputter coated with 20 nm gold/palladium in Leica AC200, and examined under a DELPHI Phenom-World microscope (EM unit, Infection Medicine, Lund University).

**Viable bacteria after continuous treatment with antibiotic fractions, and development of resistance to VAN (vancomycin), GEN (gentamicin) and RIF (rifampicin)**

Viable bacteria after continuous treatment with antibiotic fractions, and the development of bacterial resistance to VAN, GEN and RIF were determined by exposing the biofilm embedded bacteria continuously to release fractions of antibiotics from CaS/HA-VAN/GEN pellets with or without RIF from predetermined time points (Day 1, 3, 7, 14, 21, 28 and 35). Briefly, 48 h-old S. *aureus* biofilms were established on 96-well micro-titre plates as mentioned above. Biofilms were then gently washed two times with sterile PBS. The biofilms in the control and test wells were exposed to 200 $\mu$L of plain TSBG and TSBG-antibiotic fractions mixture (1:1) respectively for 16 h, followed by 8 h incubation in 150 $\mu$L of antibiotic-free TSBG. This cycle was repeated until antibiotic fractions from all time points were exposed: a total of seven days. Thereafter, each well were gently washed two times with sterile PBS. To remove the remaining biofilms from the wells, 200 $\mu$L sterile PBS was added, and wells were scraped with sterile plastic pipette tips followed by sonication of the micro-titre plate for 5 min in an ultrasonic bath (Elmasonic S 30H; Elma Hans Schmidbauer GmbH & Co. KG, Singen, Germany). After serial dilution, the number of CFUs were determined by plating on both antibiotic-free Tryptic Soy Agar (TSA), and TSA supplemented with varying concentrations of antibiotics (VAN /GEN: 4-24 $\mu$g/plate; RIF: 1-32 $\mu$g/plate). The development of resistance was tested on both *S. aureus* ATCC 25923, and *S. aureus* clinical strain P-3 whose MIC values against VAN, GEN and RIF were predetermined using MALDI-TOF MS system (Vitek MSTM; BioMe'rieux, France) (*S. aureus* ATCC 25923: VAN=2 $\mu$g/mL) GEN=0.064 $\mu$g/mL, RIF=0.008 $\mu$g/mL; *S. aureus* clinical strain P-3: VAN=1 $\mu$g/mL, GEN=0.5 $\mu$g/mL, RIF=0.008 $\mu$g/mL). Strains showing resistance to RIF were further confirmed using MALDI-TOF MS system. For each tested group of antibiotic fractions, four separate wells were used (n=4).

*Statistical Analysis*

All data was represented as mean $\pm$ SD or SEM. A student t-test was used to analyze injectability of CaS/HA-VAN/GEN with or without RIF, material degradation of the pellets, MTT assay, and to compare the differences in biofilm destruction. A student t-test or one-way ANOVA with Dunn's multiple comparisons test was used to analyze the cumulative release profile of VAN, GEN or RIF. For comparison of difference in ZOI between different groups, ANOVA with Tukey's multiple comparison was used. A $p < 0.05$ was considered statistically significant. Details of statistical analysis are indicated in each figure legend. All data processing was carried out on GraphPad Prism version 9.1.2 for MacBook (GraphPad Software, San Diego, CA, USA).

**Results:**

***Preparation and characterization of antibiotic loaded CaS/HA composites***

Pellets of all tested combinations of antibiotic loaded CaS/HA composites were characterized for the morphology and physio-chemical properties. CaS/HA pellets with either GEN or VAN were white in colour while combinations of RIF had dark orange colour (Figure 1A & B). SEM revealed that the addition of RIF to CaS/HA-VAN/GEN composites appears to have no effect either in the surface morphology or pore structure (Figure 1C & D). FTIR characterization further confirmed that the RIF was loaded onto CaS/HA-VAN/GEN composites. CaS/HA-VAN+RIF showed the same transmittance peaks at 1584 cm$^{-1}$, 1559 cm$^{-1}$, 1430 cm$^{-1}$, 1225.3 cm$^{-1}$ and 889 cm$^{-1}$ as RIF, whereas CaS/HA-GEN+RIF showed the same transmittance peaks as that of RIF at 1558 cm$^{-1}$, 1433.4 cm$^{-1}$, 1372.2 cm$^{-1}$, 1225.3 cm$^{-1}$ and 891 cm$^{-1}$, which could not be seen in only CaS/HA-VAN/GEN composites (Figure 2A & B). CaS/HA-VAN composites could be completely extruded from the syringes at all three tested time points (3, 5 and 7 min) whereas addition of RIF to CaS/HA-VAN significantly reduced the injectability, and by 5 min the composite became uninjectable (Fig.2C). The CaS/HA-GEN composites could be completely extruded from the syringes at 4 min without blocking the syringe outlet or causing obvious pressure filtration effects, and by 8 min it dropped to almost 47% (Fig.2C). By dropping the injectability to almost 12% by 6 min, a significant injectability reduction trend was observed with the addition of RIF to CaS/HA-GEN as well. At both 3 and 5 min, CaS/HA-RIF composites maintained the injectability and by 7 min it reduced to almost 11%. All the CaS/HA- antibiotics composites were completely set within 15 minutes of injection and they subjectively seemed durable and did not crumble while handled. All materials had a similar degradation rate, irrespective of the type of antibiotic added. Nearly 50% of the material degraded over a 1-month period (Fig. 2D).

***Antibiotic release profile***

In the tested CaS/HA pellets of both VAN or combination of VAN+RIF, at D-1, 3, 7 and 21, there was a significant difference in the amount of VAN released per day but overall cumulative release profile of VAN remained almost unchanged (58% and 56.7% respectively) (Figure 3 A & B). Similar cumulative release trend was observed with GEN as well (CaS/HA-GEN, 58.6% and CaS/HA- GEN+RIF, 62.6%) (Figure 3 C & D). From CaS/HA-VAN and CaS/HA-VAN+RIF, VAN concentrations above the MIC levels of *S. aureus* were obtained till day-14 (3.08 $\mu$g/mL) and 21 (2.1 $\mu$g/mL) respectively. Till study end point at day-35, CaS/HA-GEN and CaS/HA- GEN+RIF pellets eluted GEN of concentrations well above the MIC levels of *S. aureus*; 11.8 $\mu$g/mL and 9.5 $\mu$g/mL respectively. In case of RIF, an increased cumulative release profile was obtained when it combined with CaS/HA-VAN (55.5%) or GEN (60.8%) compared to CaS/HA-RIF alone (46.3%) (Figure 3 E & F). On day-35, concentrations of RIF from CaS/HA-RIF (1.94 $\mu$g/mL), CaS/HA-VAN+RIF (1.87 $\mu$g/mL), and CaS/HA-GEN+RIF (1.87 $\mu$g/mL) all found to be above the MIC levels of *S. aureus.*

***Efficacy on planktonic bacteria by continuous Kirby-Bauer disk diffusion assay***

There was a general trend that ZOI of all different composite pellets decreased over time (Figure 4A-C, Fig. 5). Out of all the tested CaS/HA pellets, antibacterial effect of CaS/HA-VAN against both S. *aureus* strains declined sharply and showed no effect by day-12. CaS/HA-GEN pellets exhibited a strong antibacterial effect until day-15 and day-3 against S. *aureus* ATCC 25923 and clinical strain P-3 respectively. There after it maintained a low but almost steady antibacterial effect till day-28. Against S. *aureus* ATCC 25923, both CaS/HA-VAN/GEN+RIF combinations showed strong antibacterial effect till day-20 but in case of clinical strain P-3, it was day-16 for CaS/HA-VAN+RIF, and day-20 for CaS/HA- GEN +RIF. Compared to pellets of all combinations, the antibacterial effect persisted for a longer time for CaS/HA-RIF pellets against both S. *aureus* ATCC 25923 (until day-26) and clinical strain P-3 (until day-25).

**Antibacterial effect of pellets after day-35**

Except CaS/HA-VAN, all other tested CaS/HA-antibiotics groups shown strong antibacterial effect at day-35 (Fig. 6). By exhibiting larger ZOI of all groups,
**[0124]** CaS/HA-GEN+RIF appears to have the strongest antibacterial effects.

**Efficacy on preformed biofilms**

Overall, there was an excellent biofilm destruction trend was observed with CaS/HA-VAN/GEN+RIF combinations than CaS/HA-VAN/GEN alone (Figure 7A-F). In comparison to CaS/HA-VAN, all tested fractions from CaS/HA-VAN+RIF shown significant destruction of S. *aureus* ATCC biofilms (Fig. 7A, D) but against clinical strain P-3 biofilms (Fig. 7B, E), only D-14 and D-35 achieved significant difference. Almost all tested fractions of CaS/HA-GEN with RIF showed significant biofilm destruction against clinical strain P-3 biofilms. Compared to CaS/HA-VAN/GEN alone, CaS/HA-RIF fractions exhibited strong antibiofilm property against both S. *aureus* ATCC and P-3 biofilms (Figure 7 C & F). SEM analysis of the preformed biofilm destruction also showed a comparable trend with that of CV staining results (Figure 8).

**Testing for development of bacterial resistance to VAN, GEN and RIF**

Following continuous treatment of S. *aureus* ATCC 25923 and clinical strain P-3 biofilms with CaS/HA-VAN/GEN/RIF alone antibiotic fractions, the number of viable CFU's obtained on antibiotic-free TSA ranged from $0-6.5\times10^7$, and $1.4-3.1\times10^8$ CFU respectively (Table 1). But with complete destruction of biofilm embedded bacteria, no viable CFU's were obtained for CaS/HA-VAN/GEN+RIF combinations, and were clearly more potent in biofilm eradication than CaS/HA-VAN/GEN/RIF alone groups.

S. *aureus* ATCC and clinical strain P-3 biofilms that had been subjected to CaS/HA-RIF alone developed resistance to RIF, and were grew on plates with 32 mg/L of RIF indicating high level of resistance. This was further confirmed using MALDI-TOF. But, none of the tested biofilms exposed to RIF in combination with VAN/GEN developed resistance to RIF. Also, no resistance to VAN or GEN were noted for any of the CaS/HA-antibiotic combinations. Bacteria in control wells were sensitive to all tested antibiotics.

**Table 1:** Viable CFU's after continuous treatment with antibiotic fractions, and development of rifampicin-resistant CFU's

| Antibiotic fractions from CaS/HA-antibiotics pellets | Bacteria tested, and total CFU before treatment | Remaining viable CFU's, median (range) | Rifampicin resistant CFU's, median (range) |
|---|---|---|---|
| CaS/HA-VAN | S. *aureus* ATCC25923, $6.5\times10^7$ | $5.9\times10^7$ ($5.6\times10^7$ - $6.5\times10^7$) | 0 |
| CaS/HA-GEN | | $2.4\times10^7$ ($1.4\times10^7$- $3.7\times10^7$) | 0 |
| CaS/HA-RIF | | $1.1\times10^7$ (0 - $4.5\times10^7$) | $1.8\times10^5$ (0 - $7.5\times10^5$) |
| CaS/HA-VAN+RIF | | 0 | 0 |
| CaS/HA-GEN+RIF | | 0 | 0 |
| CaS/HA-VAN | S. *aureus* clinical strain P-3, $2.8\times10^8$ | $1.73\times10^8$ ($1.4\times10^8$ - $1.92\times10^8$) | 0 |
| CaS/HA-GEN | | $2.54\times10^8$ ( $2.2\times10^8$ - $3.1\times10^8$) | 0 |
| CaS/HA-RIF | | $1.05\times10^8$ ($2.5\times10^2$ - $2.8\times10^8$) | $9.2\times10^7$ (0 - $1.9\times10^8$) |
| CaS/HA-VAN+RIF | | 0 | 0 |
| CaS/HA-GEN+RIF | | 0 | 0 |

**Claims**

1. A composition for use in the treatment or prevention of a bacterial infection in a subject, the composition comprising;

    i) a first material being hydroxyapatite,
    ii) a second material being calcium sulphate,
    iii) two or more pharmaceutical compounds, wherein the at least two pharmaceutical compounds are selected from a combination of vancomycin and rifampicin, or a combination of gentamicin and rifampicin,

to thereby prevent or reduce or eliminate the occurrence of bacterial resistance against said the pharmaceutical compounds.

2. The composition for use according to claim 1, wherein the first material is hydroxyapatite in any configuration, and wherein the second material is calcium sulphate in any configuration, or crystal form, or type of hydration.

3. The composition for use according to any of the preceding claims, wherein the particle size of the first material is in range of from 1 nm to 500 $\mu$m.

4. The composition for use according to any of the preceding claims, wherein the bacterial infection is in relation to bone tissue, and selected from deep bone infection (DBI).

5. The composition for use according to any of the preceding claims, wherein the two or more pharmaceutical compounds are pre-mixed or pre-soaked into the first and second material prior to administration to the subject.

6. The composition for use according to any of the preceding claims, wherein the composition is administered to the subject by injection.

7. The composition for use according to any of the preceding claims, wherein the composition is administered as a solid formulation or as a fluid or semi-fluid formulation.

8. The composition for use according to any of the preceding claims, wherein the composition is administered by injection near or into bone tissue.

9. The composition for use according to any of the preceding claims, wherein no further pharmaceutical compounds capable of treating a bacterial infection are administered after administration of the composition according to any one of claims 1-8.

10. The composition for use according to any of the preceding claims, wherein the one or more pharmaceutical compounds are released from the first and second material for an extended period of time during a period of at least 1 day, at least 3 days, at least 7 days, at least 14 days, at least 21 days, at least 28 days, or at least 35 days, or more.

11. A method of preparing a composition for use according to any one of claims 1-10, the method comprising the steps of;

> a) providing a particulate first material being hydroxyapatite and a second material being calcium sulphate,
> b) contacting/incubating/soaking two or more pharmaceutical compounds with the material in a) wherein the two or more pharmaceutical compounds are;
>
> > I) vancomycin and rifampicin in combination, or
> > II) gentamicin and rifampicin,
>
> c) separating non-bound pharmaceutical compounds from the material obtained in step b).

12. The method according to claim 11, wherein the hydroxyapatite in any configuration and the calcium sulphate in any configuration, crystal form or degree of hydration.

13. The method according to any one of the preceding claims 11-12, wherein the one or more pharmaceutical compounds are dissolved in any suitable solvent such as a physiological saline solution, water or other aqueous solution, buffered solution, or any suitable organic solvent or any mixtures thereof, and thereafter contacted with the first and/or second material.

14. The method according to any one of claims 11-13, wherein the period during which the one or more pharmaceutical compounds is contacted with the particulate first material and/or a second material, may be within range of minutes, hours or days, or 1 hour, 3 hours, 6 hours, 12 hours, 24 hours, or 48 hours.

15. The method according to any one of claims 11-14, wherein after contacting/incubating the one or more pharmaceutical compounds with the particulate first material and/or a second material, the resulting mixture may be separated from the solution or suspension comprising the one or more pharmaceutical compounds by centrifugation or filtration etc.

and wherein the resulting particulate material may be rinsed with any suitable solvent, and optionally thereafter allowed to dry.

**Patentansprüche**

1. Zusammensetzung zur Verwendung bei der Behandlung oder Vorbeugung einer bakteriellen Infektion bei einer Person, wobei die Zusammensetzung umfasst:

   i) ein erstes Material, welches Hydroxylapatit ist,
   ii) ein zweites Material, welches Calciumsulfat ist,
   iii) zwei oder mehr pharmazeutische Verbindungen, wobei die mindestens zwei pharmazeutischen Verbindungen aus einer Kombination von Vancomycin und Rifampicin oder einer Kombination von Gentamicin und Rifampicin ausgewählt sind, um dadurch das Auftreten einer bakteriellen Resistenz gegen die pharmazeutischen Verbindungen zu verhindern, zu verringern oder zu eliminieren.

2. Zusammensetzung zur Verwendung nach Anspruch 1, wobei das erste Material Hydroxylapatit in beliebiger Konfiguration ist, und wobei das zweite Material Calciumsulfat in beliebiger Konfiguration, oder Kristallform oder Hydratisierungsart ist.

3. Zusammensetzung zur Verwendung nach einem der vorstehenden Ansprüche, wobei die Partikelgröße des ersten Materials im Bereich von 1 nm bis 500 μm liegt.

4. Zusammensetzung zur Verwendung nach einem der vorstehenden Ansprüche, wobei die bakterielle Infektion in Beziehung zum Knochengewebe steht, und aus einer tiefen Knocheninfektion (DBI) ausgewählt ist.

5. Zusammensetzung zur Verwendung nach einem der vorstehenden Ansprüche, wobei die zwei oder mehr pharmazeutischen Verbindungen vor der Verabreichung an die Person vorab gemischt oder vorab in das erste und zweite Material eingeweicht werden.

6. Zusammensetzung zur Verwendung nach einem der vorstehenden Ansprüche, wobei die Zusammensetzung der Person durch Injektion verabreicht wird.

7. Zusammensetzung zur Verwendung nach einem der vorstehenden Ansprüche, wobei die Zusammensetzung als feste Formulierung oder als flüssige oder halbflüssige Formulierung verabreicht wird.

8. Zusammensetzung zur Verwendung nach einem der vorstehenden Ansprüche, wobei die Zusammensetzung durch Injektion nahe dem oder in das Knochengewebes verabreicht wird.

9. Zusammensetzung zur Verwendung nach einem der vorstehenden Ansprüche, wobei nach Verabreichung der Zusammensetzung nach einem der Ansprüche 1 bis 8 keine weiteren pharmazeutischen Verbindungen, die in der Lage sind, eine bakterielle Infektion zu behandeln, verabreicht werden.

10. Zusammensetzung zur Verwendung nach einem der vorstehenden Ansprüche, wobei die eine oder mehreren pharmazeutischen Verbindungen über einen längeren Zeitraum während einer Zeit von mindestens 1 Tag, mindestens 3 Tagen, mindestens 7 Tagen, mindestens 14 Tagen, mindestens 21 Tagen, mindestens 28 Tagen oder mindestens 35 Tagen oder mehr aus dem ersten und zweiten Material freigesetzt werden.

11. Verfahren zur Herstellung einer Zusammensetzung zur Verwendung nach einem der Ansprüche 1-10, wobei das Verfahren die Schritte umfasst zum:

   a) Bereitstellen eines partikelförmigen ersten Materials, das Hydroxylapatit ist, und eines zweiten Materials, das Calciumsulfat ist,
   b) Kontaktieren/Inkubieren/Einweichen von zwei oder mehr pharmazeutische Verbindungen mit dem Material in a), wobei die zwei oder mehr pharmazeutischen Verbindungen sind:

   I) Vancomycin und Rifampicin in Kombination, oder
   II) Gentamicin und Rifampicin,

c) Trennen nicht gebundener pharmazeutischer Verbindungen von dem in Schritt b) erhaltenen Material.

**12.** Verfahren nach Anspruch 11, wobei das Hydroxylapatit in beliebiger Konfiguration, und das Calciumsulfat in beliebiger Konfiguration, Kristallform oder Hydratisierungsgrad ist.

**13.** Verfahren nach einem der vorstehenden Ansprüche 11-12, wobei die eine oder mehreren pharmazeutischen Verbindungen in einem beliebigen geeigneten Lösungsmittel wie einer physiologischen Kochsalzlösung, Wasser oder einer anderen wässrigen Lösung, einer gepufferten Lösung oder einem beliebigen geeigneten organischen Lösungsmittel oder beliebigen Gemischen davon gelöst und danach mit dem ersten und/oder zweiten Material in Kontakt gebracht werden.

**14.** Verfahren nach einem der Ansprüche 11-13, wobei der Zeitraum, in dem die eine oder mehreren pharmazeutischen Verbindungen mit dem partikelförmigen ersten Material und/oder einem zweiten Material in Kontakt gebracht wird, im Bereich von Minuten, Stunden oder Tagen, oder 1 Stunde, 3 Stunden, 6 Stunden, 12 Stunden, 24 Stunden oder 48 Stunden liegen kann.

**15.** Verfahren nach einem der Ansprüche 11-14, wobei nach Kontaktieren/Inkubieren der einen oder mehreren pharmazeutischen Verbindungen mit dem partikelförmigen ersten Material und/oder einem zweiten Material das sich ergebende Gemisch von der Lösung oder Suspension, welche die eine oder mehreren pharmazeutischen Verbindungen umfasst, durch Zentrifugieren oder Filtern usw. getrennt werden kann, und wobei das sich ergebende partikelförmige Material mit einem beliebigen geeigneten Lösungsmittel gespült, und danach optional trocknen gelassen werden kann.

## Revendications

**1.** Composition pour utilisation dans le traitement ou la prévention d'une infection bactérienne chez un sujet, la composition comprenant :

i) une première substance étant de l'hydroxyapatite,
ii) une seconde substance étant du sulfate de calcium,
iii) deux composés pharmaceutiques ou plus, dans laquelle les au moins deux composés pharmaceutiques sont choisis parmi une combinaison de vancomycine et de rifampicine, ou une combinaison de gentamicine et de rifampicine, pour ainsi prévenir, réduire ou éliminer l'apparition d'une résistance bactérienne contre lesdits composés pharmaceutiques.

**2.** Composition pour utilisation selon la revendication 1, dans laquelle la première substance est de l'hydroxyapatite dans n'importe quelle configuration, et dans laquelle la seconde substance est du sulfate de calcium dans n'importe quelle configuration, ou forme cristalline, ou type d'hydratation.

**3.** Composition pour utilisation selon l'une quelconque des revendications précédentes, dans laquelle la taille de particules de la première substance est comprise dans la plage de 1 nm à 500 $\mu$m.

**4.** Composition pour utilisation selon l'une quelconque des revendications précédentes, dans laquelle l'infection bactérienne concerne le tissu osseux et est choisie parmi les infections osseuses profondes (DBI).

**5.** Composition pour utilisation selon l'une quelconque des revendications précédentes, dans laquelle les deux composés pharmaceutiques ou plus sont pré-mélangés ou pré-trempés dans les première et seconde substances avant leur administration au sujet.

**6.** Composition pour utilisation selon l'une quelconque des revendications précédentes, dans laquelle la composition est administrée au sujet par injection.

**7.** Composition pour utilisation selon l'une quelconque des revendications précédentes, dans laquelle la composition est administrée sous forme de formulation solide ou sous forme de formulation fluide ou semi-fluide.

**8.** Composition pour utilisation selon l'une quelconque des revendications précédentes, dans laquelle la composition est administrée par injection à proximité ou dans le tissu osseux.

9. Composition pour utilisation selon l'une quelconque des revendications précédentes, dans laquelle aucun autre composé pharmaceutique apte à traiter une infection bactérienne n'est administré après l'administration de la composition selon l'une quelconque des revendications 1-8.

10. Composition pour utilisation selon l'une quelconque des revendications précédentes, dans laquelle les un ou plusieurs composés pharmaceutiques sont libérés des première et seconde substances pendant une période prolongée pendant une période d'au moins 1 jour, d'au moins 3 jours, d'au moins 7 jours, d'au moins 14 jours, d'au moins 21 jours, d'au moins 28 jours ou d'au moins 35 jours, ou plus.

11. Procédé de préparation d'une composition pour utilisation selon l'une quelconque des revendications 1-10, le procédé comprenant les étapes de :

a) fourniture d'une première substance particulaire étant de l'hydroxyapatite et d'une seconde substance étant du sulfate de calcium,
b) mise en contact/incubation/trempage des deux composés pharmaceutiques ou plus avec la substance en a) dans lequel les deux composés pharmaceutiques ou plus sont ;

I) la vancomycine et la rifampicine en combinaison, ou
II) la gentamicine et la rifampicine,

c) séparation des composés pharmaceutiques non liés de la substance obtenue à l'étape b).

12. Procédé selon la revendication 11, dans lequel l'hydroxyapatite étant dans n'importe quelle configuration et le sulfate de calcium étant dans n'importe quelle configuration, forme cristalline ou degré d'hydratation.

13. Procédé selon l'une quelconque des revendications 11-12 précédentes, dans lequel les un ou plusieurs composés pharmaceutiques sont dissous dans n'importe quel solvant approprié tel qu'une solution saline physiologique, de l'eau ou une autre solution aqueuse, une solution tamponnée, ou tout solvant organique approprié ou tout autre mélange de ceux-ci, puis mis en contact avec la première substance et/ou la seconde substance.

14. Procédé selon l'une quelconque des revendications 11-13, dans lequel la période pendant laquelle les un ou plusieurs composés pharmaceutiques sont mis en contact avec la première substance particulaire et/ou une seconde substance, peut être comprise dans une plage de minutes, heures ou jours, ou 1 heure, 3 heures, 6 heures, 12 heures, 24 heures ou 48 heures.

15. Procédé selon l'une quelconque des revendications 11-14, dans lequel après la mise en contact/incubation des un ou plusieurs composés pharmaceutiques avec la première substance particulaire et/ou une seconde substance, le mélange résultant peut être séparé de la solution ou de la suspension comprenant les un ou plusieurs composés pharmaceutiques par centrifugation ou filtration, etc. et dans lequel la substance particulaire résultante peut être rincée avec n'importe quel solvant approprié, puis éventuellement laissée sécher.

**Fig. 1A and 1B**

Fig. 1C

Fig. 1D

Fig. 2A

Fig. 2B

Fig. 2C and D

Fig. 3

Fig. 4

Fig. 5

Fig. 6

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019206677 A
- US 2014234435 A
- CN 106943629
- KR 20160122656

**Non-patent literature cited in the description**

- **DORATI, R. et al.** Biodegradable Scaffolds for Bone Regeneration Combined with Drug-Delivery Systems in Osteomyelitis Therapy. *Pharmaceuticals,* 2017, vol. 10 (4), 96